# EUROPEAN PATENT SPECIFICATION

(11) **EP 1 866 362 B1**
(45) Date of publication and mention of the grant of the patent: **18.12.2013**
(21) Application number: 06723938.4
(22) Date of filing: 31.03.2006
(51) Int. Cl.: C08J 9/00

(54) **FOAM-LIKE PREPARATION AND PROCESS FOR THE PRODUCTION THEREOF**
SCHAUMARTIGE ZUBEREITUNG UND HERSTELLUNGSVERFAHREN DAFÜR
PREPARATION DE TYPE MOUSSE ET SON PROCEDE DE FABRICATION

(30) Priority: 01.04.2005 DE 102005015321
(43) Date of publication of application: 19.12.2007
(73) Proprietor: Schwan-STABILO Cosmetics GmbH & Co. KG, 90562 Heroldsberg (DE)
(72) Inventor: ZECH, Christina, 86916 Kaufering (DE); BUMILLER, Daniela, 90411 Nürnberg (DE)
(74) Representative: Leissler-Gerstl, Gabriele
(86) International application number: PCT/EP2006/002978
(87) International publication number: WO 2006/103102

(56) References cited:
- US-A- 5 720 943
- US-A1- 2005 069 566
- US-B1- 6 491 931

## Description

The invention concerns a foam-like preparation, in particular for use for cosmetic materials, and a process for the production thereof.

Foam-like cosmetic products, frequently also referred to as 'mousse' are any products, for example in the form of shaving foam, foam masks, hair care products or hair setting products. With those products the foam is generally produced immediately prior to application by a composition being caused to foam up by added foaming propellent gas. Foam-like products are advantageous for the reason that they can be applied more easily due to their greater volume, but at the same time they have to contain fewer ingredients by virtue of their lower density. The foam-like products which are known for that purpose are mostly packaged in the form of aerosols in compressed air packs and foaming is then effected by means of the added propellent gas. Foamed products are also known in the field of foodstuffs. Dinitrogen monoxide is frequently used here for foaming purposes. Thickening agents often have to be used for the stabilisation of foam products of that kind. It will be noted however that foam products suffer from many problems. Thus for example in the production of cosmetic products in the form of emulsions, gels or oleogels, irregular structures and fluctuations in density in the filling procedure can occur due to included air. As the products are filled into containers on the basis of volume, the filling mass is influenced thereby.

A further disadvantage of included air is the oxygen component which can lead to changes in quality in a preparation. Thus, cosmetic preparations can be detrimentally affected as a consequence of rancidity or due to the formation of organic peroxides and markedly perceptible changes in smell which occur as a consequence thereof.

As foaming is often considered to be disadvantageous in the case of many compositions which are filled into containers and stored in that filled form, steps are taken to prevent foaming or the inclusion of air bubbles. For that reason for example in the production of cosmetic preparations operation is frequently implemented in vacuum vessels and the emulsion which is still liquid in the hot condition or the precursors thereof are subjected to an air removal procedure in order to remove gases dissolved therein.

When foam-like cosmetic products or cosmetic products containing air bubbles are introduced into containers, that can also involve adverse aesthetic effects. If included air bubbles escape in the course of storage, the product collapses and becomes unsightly. Such a product cannot therefore be sold in a transparent pack.

The attempt has therefore been made to provide stable foam-like products. Thus for example an alcohol-free foam carrier for cosmetic or pharmaceutical uses is described in US No 2005/0069566. That foam carrier is intended to produce a stable structure and contains hydrophobic solvents but no alcohol. The foam carrier comprises a gelling agent which is an amphiphilic copolymer, in combination with a polymer film-forming agent which can be for example an acrylate polymer. The composition referred to as the foam carrier is produced from water, hydrophobic solvents, tenside, gelling agent and propellent gas, whereby a stable emulsion which has good storage capability is produced.

The known foam-like products however suffer from disadvantages. Thus the products can be dispensed only by way of aerosol containers. In addition the foam cannot be mechanically loaded and stressed, that is to say it breaks down upon being rubbed.

Now the object of the invention is to provide a foam-like composition which affords a stable foam without the need to use aerosol containers or propellent gases, which leads to a high-quality product of reliable quality and the density and gas content of which can be predetermined or adjusted.

A further object of the invention is to provide a preparation which can be satisfactorily filled into containers even at elevated temperature as that counteracts microbiological contamination.

That object is attained by a preparation in foam form, as defined in claim 1, and a process for the production thereof. The preparation according to the invention is distinguished by a stable structure which is formed by the specific combination of ingredients according to the invention, which can stably hold gases introduced in manufacture and which after the incorporation of the gas affords a product of low density and at the same time in stable and homogeneous form.

The advantageous properties are achieved by the use of a system comprising a continuous phase and a thickener, wherein the thickener in the continuous phase has a miscibility gap with a lower critical temperature of at least 45°C.

It was surprisingly found that, when using a system comprising a continuous phase and a thickening agent in an emulsion, a stable foam can be produced when the thickening system has a miscibility gap having a lower critical temperature which is above the temperature of use. In that respect the thickening system can be formed in an emulsion both in the aqueous phase and in the oil phase. In order more precisely to describe the present invention, production of an oil-in-water emulsion (O/W emulsion) is described. The same description however equally applies to a water-in-oil emulsion (W/O emulsion).

Reference is made to a miscibility gap when two components mix in a given temperature range but outside that temperature range form two phases. There is then the 'critical solution temperature' which separates the two ranges. The inventors surprisingly found that, when using a system comprising a continuous phase and thickening agent, which has a miscibility gap with a lower critical temperature, it is possible to produce very stable foam-like emulsions if the critical temperature is above the temperature of use. Without being bound by a theory, it is assumed that, above the critical temperature, the phases separate or the thickening agent precipitates, while below the critical temperature the thickening agent is dissolved and can perform its function of structure formation. By virtue of that phenomenon, it is possible to produce above the temperature of use a foam-like emulsion which is stabilised below the critical temperature by the thickening agent which is then structure-imparting, and which contains gas bubbles, that are evenly distributed and all have a similar size.

If an emulsion is formed from two phases of which one is a thickening system with a miscibility gap and if those two phases are combined above the critical temperature, the two phases can be very well mixed. If then the temperature is reduced below the critical point with agitation, the thickener is dissolved and forms its structure which provides that air introduced by the agitation procedure or introduced gas no longer escapes but is included therein. Coalescence of the introduced air bubbles is prevented by the additional marked rise in viscosity which is caused by the temperature of the emulsion falling below the gel network temperature during the cooling phase. In that way it is possible to introduce a large proportion of gas into the emulsion and to form a foam-like composition which, after cooling to ambient temperature (23 +/- 2°C), if it is stored at ambient temperature or below, remains stable during a storage time of at least 36 months and often virtually unlimitedly.

The advantages of a foam-like preparation of that kind is that the emulsion is light and homogeneous, stable in respect of storage and good to apply. Preparations of that kind are particularly suitable in the field of cosmetics and foodstuffs, where voluminous masses are desired for many applications. The product according to the invention can also be used for all applications in which foam-like products of the state of the art are hitherto used in the cosmetic field, and it affords advantageous properties. The composition according to the invention can also be used well in the foodstuffs field in order to produce lighter products, for example calorie-reduced products. Examples are cream-like or creamy products or yoghurts and puddings.

Essential ingredients of the foam-like preparation according to the invention are a hydrophilic and a hydrophobic phase for forming the emulsion and the use of the thickening system. A further important component is a structure-forming agent.

The preparation according to the invention is in the form of an emulsion and can be both a W/O and also an O/W emulsion. The thickening system is respectively formed in the continuous phase, that is to say in the aqueous phase in the case of an O/W emulsion and in the oil phase in the case of a W/O emulsion. The system comprising the continuous phase and the thickening agent has a miscibility gap which occurs above the temperature of use. In that respect the critical temperature must be above the temperature of use so that the thickening effect can occur at the temperature of use. On the other hand the critical temperature may not be excessively high as otherwise the components would have to be heated to an excessively high temperature in the production process, and that is harmful for many ingredients. Therefore, in the case of cosmetic products, the critical temperature is more appropriately above body temperature, in particular above 45°C, and on the other hand does not exceed 100°C. Preferably the critical temperature is between 35 and 80°C.

In a preferred embodiment the thickening system is present in the aqueous phase and comprises water or an aqueous medium such as a water-alcohol mixture, and the thickening agent. In that case the thickening agents used in cosmetic or foodstuffs-chemical applications are suitable as the thickening agent, if they have a critical temperature in the desired range. Cellulose derivatives, in particular methyl cellulose and hydroxypropyl cellulose, the critical temperature of which is in the desired range, have proven to be preferred thickening agents. The amount of thickening agent in the thickening system is from 0,5 to 5 % by weight.

For an O/W emulsion, the continuous phase is water or an aqueous medium. In order to increase the workability, preferably an aqueous medium is used, which comprises water and smaller proportions of hydrophilic solvents such as monovalent or polyvalent alcohols. Short-chain monovalent alcohols such as in particular ethanol and propanol, short-chain polyvalent alcohols, in particular diols, such as propane diols, butylene glycols or hexylene glycols, are suitable. By way of example mention may be made of propane-1,2-diol, propane-1,3-diol, butylene-1,2-,1,3- and 1,4-glycol, hexylene-1,2-, 1,3-, 1,4-, 1,5- and/or 1,6-glycols, dipropylene glycol, tripropylene glycol, glycerine, diglycerine, triglycerine and polyvalent alcohols such as sorbitol, mannitol, xylitol, alditol and so forth. The critical temperature of the system can be influenced by using an organic solvent acting as a co-solvent. The solvent can therefore optionally be used to adjust the critical temperature into a suitable range. The amount of solvent therefore depends both on the solubility in water, the solution capability for the ingredients and also the influence on the critical temperature, and is therefore preferably used in a proportion such that all properties are in a desired range. Usually the aqueous phase contains solvent in a proportion of 0 to 15% by weight, preferably 1 to 5% by weight.

The proportion of thickening agent in the thickening system depends inter alia on the swelling capability and the thickening capability of the compound used. If a cellulose derivative, in particular methyl cellulose or hydroxypropyl cellulose which each have a very strongly swelling action is used in an aqueous system, the proportion in the range of 0.5 to 5% by weight with respect to the weight of the aqueous phase. Preferably the proportion of the thickening agent is 2.0 to 3.5% by weight with respect to the aqueous phase. If other thickening agents are used, the thickening effect of which is less, the proportion can be correspondingly increased.

The second phase of the emulsion also contains a structure-forming agent to obtain a structured stable end product. In the case of an O/W emulsion, hydrophobic oil-soluble agents are considered as the structure-forming agent, as are generally used in the field of cosmetics and foodstuffs. In a preferred embodiment, if the preparation according to the invention is used in the cosmetic field, the oil phase contains wax as a structure-forming agent. The waxes which are usually employed can be used here. The wax forms the main ingredient in the oil phase and is preferably contained in a proportion of 5 to 40% by weight, particularly preferably 10 to 30% by weight, with respect to the total weight of the preparation.

The oil phase can be made up of fat-like, oil-like and/or wax-like substances of natural and/or synthetic origin or mixtures thereof, which can be liquid, pasty or solid at ambient temperature. Frequently a combination of at least one wax and at least one oil is used to adjust the optimum consistency. A combination of waxes is also advantageous. Volatile oils can be added in order to prevent sticking or to enhance drying of the texture.

Preferably at least one wax is used, which is solid at ambient temperature. Particularly preferred are such waxes with a dropping point of 50 to 200°C. Waxes with a dropping point below 50°C can give rise to problems in terms of storage capability and waxes with a dropping point above 200°C are sometimes difficult to work.

The waxes which are usually employed in cosmetics are suitable as the wax component, in particular vegetable waxes such as carnauba and candellila waxes, ouricuri wax, Japan wax, cotton wax, rice wax, flower wax, hydrated jojoba oil; animal waxes such as beeswaxes and modified beeswaxes, inter alia cera bellina, lanolin waxes and insect waxes; mineral waxes such as paraffin waxes, microcrystalline waxes, montan waxes as well as ozocerite, as well as synthetic waxes such as Fischer-Tropsch waxes, wax-polymer hybrids, such as PVP hexadecane copolymer, polyethylene waxes, silicone waxes and acelaic acid diolelyl ester, acelaic acid dibehenyl ester, behenyloleate, behenylbehenate, cetyl palmitate and mixtures of all listed waxes. Animal waxes and synthetic waxes as well as in particular mixtures thereof are more preferably used.

A fat or oil can be contained in the oil phase in order to adjust the viscosity of the material and to impart maleability thereto. Both animal and also vegetable fats and oils can be considered for that purpose, which can also be used in hydrated or modified form. In particular triglycerides, modified triglycerides, oligomeric and polymeric glycerine derivatives which are esterified for example with one or more fatty acids are considered as fats in this respect. Usually vegetable, animal, mineral or synthetic oils are used as the oils here. Natural and care oils are particularly suitable for cosmetic products. Examples are set forth hereinafter, without this list being exhaustive: rapeseed oil, sunflower oil, sesame oil, peanut oil, thistle oil, coconut oil, hydrated coconut oil, castor oil, hydrated castor oil, beef tallow, isopropyl myristate, isopropyl palmitate, isopropyl stearate, butyl stearate, isobutyl stearate, isostearyl isostearate, oleyl oleate, jojoba oil, caprylic/capric triglycerides and similar synthetic triglycerides, paraffin oil, polybutene, squalane, squalene and mixtures thereof, synthetic esters, silicone oils, in which case here both volatile and also non-volatile oils can be considered, in particular dimethicone, phenyl trimethicone and cyclodimethicone as well as volatile and non-volatile isoparaffins, fluorine oils, volatile ethers, esters and ketones and copolymers of the above-mentioned. Mixtures of those constituents are preferred. Thus frequently both a non-volatile fat or oil and also a volatile oil is contained in order to impart various properties. In an embodiment the oil phase contains volatile constituents, in particular synthetic esters, silicone oils, dimethicone, phenyl trimethicone and cyclomethicone and isoparaffins in a concentration of up to 20% with respect to the oil phase.

Mixtures of waxes, fats and oils are used in such amounts that the desired properties such as texture and viscosity are achieved. The respective amounts and mixtures to be used are basically known to the man skilled in the art and do not need to be described in greater detail here.

Preferably the oil phase is used in such a proportion that it forms 0.5 to 70%, preferably 5 to 50%, more preferably 15 to 30%, of the final composition, in which respect the percentages, like all details in the description unless otherwise stated, refer to weight. Within the oil phase the proportion of the wax or waxes is preferably between 20 and 100% and more preferably between 60 and 95%.

The proportion of fat or oil in the oil phase is usually in such a range that the mass is of the desired consistency. Normally the proportion of fats and oils in the oil phase is between 0 and 80%, depending on the materials used, with the lower range being preferred.

An emulsion is formed from the aqueous and the oil phases, an emulsifier generally being added for that purpose. If used, the emulsifier is preferably added to the oil phase. It was surprisingly found that particular properties with the present preparation can be obtained if an emulsifier is used, whose HLB value is between 3 and 8. Non-ionic emulsifiers were found to be particularly suitable. Examples thereof are glycerides, that is to say glycerine esters of long-chain fatty acids, with mono- and diglycerides or a mixture thereof being particularly suitable. In a preferred embodiment of an O/W emulsion an O/W emulsifier is added to the oil phase and leads to the structure which is desired in accordance with the invention. A glyceryl stearate is preferred, more preferably glyceryl monostearate.

The emulsion according to the invention, besides the above-discussed essential constituents, can contain still further ingredients in order to adjust desired properties which are discussed hereinafter.

An acid or base or a buffer system can be added to the aqueous phase to adjust the pH-value. Acids and bases which are suitable for that purpose are known in the field of cosmetics and foodstuffs. Amine derivatives such as tromethamine (tris(hydroxymethyl) aminomethane) have proven to be particularly suitable. The pH-adjusting agent is used in such an amount that the desired pH-value is achieved. If the product according to the invention is a cosmetic product the pH-value is usually between 5 and 8, particularly preferably between 7 and 7.5. In the case of a foodstuff the pH-value depends on the respective product and for example for a salad dressing is in an acid range while for a dessert product it is in the range of between 5 and 8.

In order to facilitate distribution of the thickening agent in the aqueous phase it is optionally possible to add a co-solvent or a dispersing aid. The co-solvent also serves to prevent lump formation when using a thickener in powder form. Suitable agents are known to the man skilled in the art. Low alcohols or diols have proven to be suitable. They behave in relation to the thickener as a non-swelling agent. When water is added the alcohol molecules are gradually replaced by water and homogeneous swelling of the solid particles of the thickener, which are isolated in the non-swelling agent, takes place without lump formation. Butane diol has proven to be particularly suitable when using cellulose derivatives as the thickening agent.

In order to influence the structure of the product according to the invention it is possible to add polymers which are suitable for that purpose. Preferably polymeric film-forming agents as are well-known are used for cosmetic preparations. The term 'polymeric film-forming agents' is used to denote those polymers which, optionally after removal of a dispersing agent or solvent, form a film, preferably a flexible film. Preferably the film-forming system used is one which is usually formed by a polymer or a plurality of polymers which is or are in dispersion or dissolved in a medium, wherein the polymer or polymers form or forms a film upon removal of the medium, that is to say the solvent or dispersing agent. Depending on the kind of polymer and the medium the solids content is usually between 10 and 70%, preferably between 20 and 40%. In the present application any system comprising a film-forming agent and medium (also referred to as solubilising agent, solvent or dispersing agent) is identified generally as a dispersion in order to obviate obscurities as the transitions between solution and dispersion are fluid, in the field of polymers. The expression 'dispersion' therefore also embraces solutions. Systems are also included, in which the film-forming polymer was solubilised for example by the addition of an acid or base. The medium is preferably water.

Film-forming polymers are known per se and all those which are usually employed for cosmetic preparations can be used. The film-forming polymer can be a polycondensate, a polyaddition product, a radically or ionically produced polymer or a polymer of natural origin. Film-forming systems which are particularly suitable for the preparation according to the invention contain anionic, cationic, non-ionic and/or amphoteric polymers from the class of polyurethanes, polyureas, polyesters and polyethers as well as derivatives thereof, PVP, polyamides, vinyl, acryl and methacryl polymers and copolymers, epoxy esters, silicone polymers and copolymers and hybrid polymers formed from the above-mentioned polymers. The film-forming system is usually present in the form of a dispersion, with aqueous systems being preferred.

Particularly preferred film-forming agents are polyurethanes and polyurethane copolymers such as for example polyurethane-acrylic acid copolymers, polyurethane-polyvinyl pyrrolidones, polyester polyurethanes, polyether polyurethanes, polyureas, polyester amides, polyesters with fat chain, polyamides, epoxy ester resins and/or polypropylene glycol-maleic ester copolymers. Many of those polymers are commercially available. A further preferred group are acryl polymers, acryl/styrene copolymers, acryl/vinyl copolymers and acryl/silicone copolymers as well as combinations of nitrocellulose and acryl polymer. Hybrid polymers can also be used in accordance with the invention. Polymers of natural origin which can optionally also be present in modified form such as for example polysaccharide and cellulose derivatives are suitable. A mixture of two or more of the listed polymers can also be used, in which case the mixture is so formed that the desired properties are achieved. The products permitted by the CTFA, in particular polyurethane-1 to polyurethane-13, are also particularly suitable.

In particular polyacrylates, polymethacrylates, polyurethanes, polyesters, polyether urethanes, polyester urethanes and polyureas are suitable. Polyurethanes and copolymers thereof, in particular polyether urethanes and polyester urethanes, have proven to be particularly suitable. Products which are commercially available under the trade mark name Avalure^{®} are particularly suitable, in particular Avalure^{®}-UR products such as Avalure^{®} UR 450.

The amount of the film-forming agent can be in a range of 0 to 50% by weight, preferably 0 to 30% by weight.

Emulsions are microbiologically susceptible by virtue of their water component. As microbial contamination in use might be possible , preserving agents are usually added. The appropriate preserving agents in the field of cosmetics or foodstuffs can be used here in per se known amounts. Phenoxyethanol has proven to be particularly suitable. A preferred amount of use of such a preserving agent is in the range of 0.5 to 1.5% by weight with respect to the weight of the overall composition.

Further ingredients to be mentioned, which are used in particular when employed in the cosmetic region, are colouring agents, in particular inorganic and/or organic dyes and/or pigments. Pigments, like also dyes, serve to impart colour to the composition. They are therefore used in many different ways in decorative cosmetics. Suitable pigments are the pigments which are generally employed in cosmetics and which are used in the amounts that are usual per se and which are suitable for achieving the aesthetic impression.

Examples of pigments are cerium oxide, black, red, yellow and brown iron oxides, chromium oxide, chromium hydroxide, manganese violet, ultramarine, chromium oxide green, chromium hydroxide green, carbon black, titanium dioxide, zinc oxide, barium sulphate, talc and kaolin. Examples of organic colouring agents are lakes of organic colouring agents with aluminium, barium, potassium, calcium, zirconium or strontium, carmine and other colouring substances well known to the man skilled in the art. Pearlescence pigments may also be contained therein. The colouring ingredients are used in the proportion usually employed for cosmetics, the amount involved being selected in dependence on the nature of the colouring agent and the desired effect. Preferably the proportion of the colouring ingredients is in a range of up to 30%, particularly preferably 2 to 20%.

Soluble dyes which are known per se to the man skilled in the art are also suitable. The following may be mentioned as examples: C.I. pigments Red 57:1, Red 49:1, Red 48:1, Red 52:1, Red 41, Red 3, Red 23, Red 112, Red 169, Orange 16, Orange 5, Violet 1, Violet 23, Green 7, Blue 61, Blue 62, Blue 15:1, White 6, Yellow 3, Yellow 13, Yellow 55, Yellow 126, FD & C Red 3, D & C Red 6, 7, 30, 36, 40, FD & C Yellow 5, 6,: D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green 5 (CI 61 570), D & C Yellow 10 (CI 77 002), D & C Green 3 (CI 42 053), D & C Blue 1 (CI 42 090), and fluorescent colouring agents such as C.I. 45380, 45370:1, 47000, 47005, 42090, 60730 or 61570. The lists are only by way of example and in no way conclusive. Organic colouring agents can also be used attached to carriers. The appropriate colouring agents are usually selected depending on the respective desired colour shade, colour depth and also the extent to which they are allowed in the field of cosmetics by national or regional legislation. In addition, suitable colouring agents can be incorporated for achieving particular colouring effects, for example colouring effects which are visible in black light, that is to say UV light. Colouring agents of that kind are commercially available, for example immobilised on plastic matrices.

Fluoresceines, quinoline dyestuffs, triphenylmethane dyestuffs and anthraquinone dyestuffs can also be named as dyestuffs which produce luminescent, in particular fluorescent, phosphorescent effects or effects which light under UV light. They can be attached to a polymer matrix which is also known as 'polyester-3' to the man skilled in the relevant art. Plastic matrices of that kind can be produced for example by the reaction of ethylene glycol, cyclohexane dimethanol, terephthalic acid, isophthalic acid and norbornane diamine in the presence of the dye. Colouring agents belonging to the class of flat pigments can also be incorporated.

Preferably food dyes or food colouring as are generally known are used for foodstuffs, in the per se known amounts.

The proportion of the colouring agents is usually in a range of between 0.5 and 25% by weight and is dependent on the nature of the colouring agent used. For pigments the range is generally between 3 and 25% by weight, particularly preferably 15 to 18% by weight. Soluble colouring agents are generally used in smaller amounts, the proportion thereof is therefore preferably between 0.5 and 18, particularly preferably between 3 and 18% by weight.

Further ingredients which can be contained in the composition according to the invention are for example moisturising agents for keeping it moist and softeners such as glycerine, propylene-1,2-glycol, butylene glycols, hexylene glycols, sodium PCA, urea and mixtures thereof. Anti-oxidants and stabilising agents can be added in per se known manner and in the per se known amounts. Depending on the respective product involved it is possible to add flavouring substances, fragrances and sweeteners. Perfumes are possibly added for cosmetic uses in the per se known usual amounts.

In order to adjust the consistency of the preparation according to the invention it is possible to add fillers such as talc, kaolin, polyethylene or nylon powder or fibres. Suitable fillers are also mica, crushed or spray-dried or precipitated polymers such as acrylate; Teflon powder, polyethylene powder, silicone cross polymers and other cross polymers. The preparation preferably has a dynamic viscosity which is in a range of 1 to 10 000 mPa.s, preferably 1 to 3 500 mPa.s.

If the preparation according to the invention is used as a sun protection agent the light protection compounds which are usually employed can be contained therein, in the per se known amounts, such as for example 4-methylbenzylidene camphor, isoamyl-p-methoxy cinnamate, octyl-p-methoxy cinnamate, 3-benzylidene camphor, octyl salicylates, benzophenone-4, octyl dimethyl PABA and mixtures thereof. The amounts used depend on the one hand on the desired purpose of use and on the other hand also on the statutory regulations.

The specific composition and structure gives rise to a foam-like product having gas bubbles which afford a pore-like structure. In that respect, depending on the respective proportion of the gas phase and the structure-producing agents, smaller or larger bubbles can be produced, for example those of a mean size range of 0.1 to 0.5 mm but also micro-bubbles in a mean size range of 0.005 to 0.1 mm. Adjustment of the bubble size is effected by way of the proportion of the gases and the thickening agents but also by virtue of the physical conditions in production of the emulsion and the subsequent processing steps.

The foam-like preparation according to the invention contains gas bubbles or air bubbles. For most purposes it is preferable for the incorporated gas to be air. In order to achieve special effects however it is also possible to use an inert or protective gas. Thus for example if the preparation contains easily oxidisable substances, it is possible to use a protective gas such as nitrogen, helium, neon, argon, krypton, xenon, carbon dioxide or dinitrogen monoxide. Hydrocarbon gases such as propane, butane or ether such as dimethyl ether can also be incorporated. The incorporation of pure oxygen, ozone, or air mixtures with an enriched oxygen content is also possible if the product is to make more oxygen available.

The size of the air bubbles can be measured with methods which are well-known to the skilled artisan. The size can for example be determined with a microscope or by freeze fracturing.

The final foam-like product of the present invention can contain a predetermined amount of gas. It is preferred that the ratio of gas that is introduced into the composition and is contained in the final product is in a range of 1 to 90 % by volume. In other words up to 90 % of the volume of the final product can be gas. Preferably 15 to 70 % of the final product are comprised of gas and more preferably 20 to 40 % by volume. For the sake of good order it should be mentioned that except for the gas content the percentages given in the description with regard to the ingredients refer to the weight of the composition without gas.

The preparation according to the invention can be processed to afford many different kinds of products. Cosmetics such as care and decorative products can be named as examples. Care products are creams, ointments, lotions, for example body lotion, anti-ageing products, day and night creams, anti-wrinkle creams, eye care products; products for hair care such as conditioner, packagings and styling products. Makeup products which are considered include products which are applied to the skin and skin appendages, in particular mascara and hair dyes. Products such as lip rouge, blusher, eyeshadow, makeup, foundations, products to deal with rosacea, tattoos or special camouflage products are also to be mentioned.

There are also many uses for the preparation produced in accordance with the invention in the foodstuffs field. Thus they can be used in the form of mayonnaise products, salad dressings, salad creams, fresh cheese-like products, pudding-like and cream-like products.

The structure to be achieved in accordance with the invention gives rise to products whose density is lower than in the case of comparable preparations. In accordance with the invention it is possible to adjust the density of emulsions in a range of 0.3 to 0.95, preferably 0.45 to 0.85, particularly preferably 0.7 to 0.8 mg/mm³.

An advantage of the present invention is that a defined amount of gas can be incorporated by setting the appropriate conditions and that specific effects can be achieved by selection of the gas.

In order to obtain the products according to the invention involving a specific and special structure, there is also provided a process for the production of a foam-like preparation. A preferred process is defined in claim 23.

To produce the preparation according to the invention and in particular to achieve the advantageous structure for the foam-like product, the procedure adopted is as follows. A continuous phase is prepared, in which the thickening agent is dispersed. As stated above the continuous phase can be formed for example from water, optionally in combination with co-solvents. The thickening agent is distributed in the continuous phase to form the thickening system. A dispersing aid can optionally be added to ensure good distribution. Apparatuses for dispersion of the thickening agent are known. It is possible to use for example apparatuses such as a dispersing disc, an Ultraturrax, a toothed ring, a colloid mill or the like. If cellulose derivatives are used as the thickening agent the use of mixers which produce a heavy mechanical loading or high shear such as an Ultraturrax should rather be avoided.

In a second step the discontinuous component of the preparation is prepared. For that purpose the structuring agent is added to the discontinuous phase, optionally with heating. In a preferred embodiment the wax and the other oily constituents are melted. The constituents are heated to such an extent that they fuse. In general that is the case at a temperature in the range of 60 to 95°C, preferably 70 to 90°C.

An emulsifier, preferably an emulsifier with an HLB value of 3 to 8 can then be added to the oil phase.

The discontinuous phase is then added to the continuous phase and agitation is effected until a homogeneous emulsion is produced. It is then optionally possible to add to that emulsion, the temperature of which must be above the critical temperature of the thickening system, the temperature-sensitive constituents such as for example polymeric film-forming agents. The emulsion is then agitated with the incorporation of air or a gas component, whereby it cools down below the critical point. Preferably the agitation is adjusted such that 1 to 90 % by volume of gas, for example air, more preferably 15 to 70 % by volume and most preferably 20 to 40 % by volume of air or a gas component are incorporated in the emulsion. The result is that the thickening agent is dissolved in the continuous phase and develops its structure. The structuring agent present in the discontinuous phase also develops its structure and additionally fixes the gas bubbles produced.

An O/W emulsion is produced in a preferred embodiment. For that purpose an aqueous phase is provided, in which the thickening agent is dispersed. As stated above the aqueous phase can be formed from water, optionally in combination with co-solvents. The thickening agent is distributed in the aqueous phase. It is optionally possible to add a dispersing aid in order to ensure good distribution. The further watersoluble components are added to the aqueous phase, with the exception of the polymeric film-forming agent if such is used.

It has proven to be advantageous if the gel network temperature of the emulsion is below 65°C, preferably between 40 and 65°C. That results in a foam-like product which retains its porous structure even when it is stored at ambient temperature over a prolonged storage time. The gel network temperature can be influenced by the choice of the nature and amount of the solvents and thickening agents. What is essential to the process according to the invention are heating the emulsion to a temperature above the critical point, and the incorporation of gas, in particular by agitation, while the emulsion slowly cools down to below the critical temperature. The gas bubbles are stabilised in that way. In order to achieve a foam which is very well stabilised, the gas bubbles should be so small that no coalescence occurs. That is the case with the above-specified size which can be implemented by adjusting the agitation speed.

By virtue of the fact that the emulsion is heated to a temperature above the critical temperature of the thickening agent, the thickening agent, as it is not in solution, cannot perform its thickening action there and the viscosity falls. If that low-viscosity emulsion is agitated, air is very easily incorporated. As soon as the temperature falls below the critical temperature again however, the structure is again formed and the incorporated gas bubbles are stabilised and can no longer flow together and escape. The structure which is advantageous in accordance with the invention is produced by virtue of that.

The process for the production of the preparation according to the invention is highly simple as it is possible to mix together the individual constituents and foam them. The size of the pores can be adjusted by adjusting the rate at which the emulsion is agitated upon cooling and, as described, by the choice of the structure-forming agents and the emulsifiers.

The resulting material has many advantages. Thus air can be incorporated in a defined fashion and also liberated in a defined fashion. Instead of air it is also possible to use protective gases or reactive gases. In an embodiment in which the product contains unsaturated fatty acids, instead of air it is possible to add nitrogen which protects the unsaturated acids from oxidation.

The process according to the invention produces a highly homogeneous product in which the pores are distributed uniformly over the mass. It is possible to incorporate a high amount of air or gas, such as up to 90 % by volume, preferably 25 to 40% by volume of air or gas so that the density of the resulting product can be greatly reduced. Thus for example it is possible to obtain a mascara mass whose density is in the range of 0.7 to 0.9, in comparison with usual mascara masses whose density mostly is considerably above 1.

The product according to the invention is light and at the same time has volume, which is perceived to be advantageous. If the product is used for a cosmetic composition, in particular for a mascara mass, the gas bubbles are also at least partially still retained upon application and after drying the result is a coating which is admittedly firm but highly elastic, porous and voluminous and which has very good adhesion over a long period, which does not transfer on to surroundings and which does not flake off. If the product is used as mascara therefore it is possible to achieve an application which emphasises the eyelashes without a 'panda bear effect' then occurring due to colour transfer on to surroundings. It has been found that pigments can also be added without problem to the preparation according to the invention. Usually the incorporation of pigments entails problems as at their surface they adsorb gases which are incorporated into the material and which in the case of usual preparations have to be removed therefrom again prior to manufacture. In the case of the preparation according to the invention that adsorption of gases leads to improved processing. It is therefore possible to use pigments without a coating; coated pigment particles are also suitable.

### Examples

### Example 1

Set out hereinafter is a formulation for a mascara preparation. The amounts specified relate in each case to parts by weight.

| | |
|---|---|
| Distilled water | 45.0 |
| Avalure^{®} UR 450 | 20.7 |
| Pigments | 10.0 |
| Cetearyl alcohol | 5.0 |
| Candelilla wax | 3.0 |
| Glyceryl stearate | 2.0 |
| Butylene-1,3-glycol | 1.5 |
| Ethanol | 1.5 |
| Hydroxypropyl cellulose | 1.0 |
| Polybutene | 1.0 |
| Tromethamine | 0.3 |
| Phenoxyethanol | 1.0 |

A foam-like preparation was produced from the above-specified ingredients, as follows. Firstly the water was placed in a vessel and hydroxypropyl cellulose, tromethamine and butylene glycol and ethanol were added with agitation. A dispersing disc was used for agitation purposes and the batch was agitated until the hydroxypropyl cellulose was homogeneously distributed. In a second vessel candelilla wax and beeswax were heated to 80°C, in which case they fused. Cetearyl alcohol was added and then glyceryl stearate. The wax melt and phenoxyethanol were then added to the water phase and agitated with a dispersing disc until a shiny emulsion had been formed. The emulsion was kept at a temperature of about 60°C and Avalure^{®} UR450 was added with agitation. The heat source was then removed and further agitation effected, with the emulsion slowly cooling down. Due to vigorous agitation with the dispersing disc the emulsion foams up and at the same time slowly cools down. Due to the foaming effect 25% by volume of air was incorporated into the emulsion. Upon cooling a structure was formed so that the foam no longer collapsed but remained stable. The material obtained is suitable as a basic material for mascara.

### Example 2

Cosmetic materials can be produced with the constituents specified hereinafter in the concentration ranges specified in relation thereto, as described in Example 1.

| | |
|---|---|
| Water | 30 - 50% |
| TEA or AMP | 0.1 - 3% |
| Methylcellulose | 1 - 5% |
| Butylene glycol | 2 - 4% |
| Ethanol | 2 -10% |
| Preserving agent | 0 - 1 % |
| Coloring agents | 2 - 20% |
| Glyceryl stearate | 2 - 3% |
| Beeswax | 7 - 13% |
| Jojoba wax | 4 - 6% |
| Gum arabic | 1 - 3% |
| Stearic acid | 5 - 7.5% |
| Polymeric film-forming agent | 1 - 20% |

## Claims

1. A foam-like cosmetic preparation in the form of an O/W emulsion comprising a continuous phase, a discontinuous phase, and a gas, wherein the continuous phase comprises a thickening system being formed by water or an aqueous medium and a thickening agent, wherein the proportion of the thickening agent in the thickening system is 0.5 to 5% by weight, and wherein the discontinuous phase comprises a structuring agent, wherein the thickening system has a miscibility gap with a lower critical temperature between 35 and 80°C.

2. A preparation according to claim 1, wherein the aqueous medium is a mixture of water and a hydrophilic solvent.

3. A preparation according to claim 1 or 2, wherein the aqueous medium is a mixture of water and monovalent alcohols selected from ethanol and propanol, or polyvalent alcohols selected from propane diols, butylene glycols, hexylene glycols, dipropylene glycol, tripropylene glycol, glycerine, diglycerine, sorbitol, mannitol, xylitol, and alditol.

4. A preparation according to claim 1, wherein the thickening system is formed by an aqueous medium and a cellulose derivative.

5. A preparation according to one of the preceding claims, wherein the discontinuous phase is an oil phase which is made up of fat-like, oil-like and/or wax-like substances.

6. A preparation according to one of the preceding claims, wherein the oil phase comprises 20 to 100% wax and 0 to 80% oil and/or fat.

7. A preparation according to one of the preceding claims, wherein an emulsifier is contained in the emulsion.

8. A preparation according to one of the preceding claims, wherein an emulsifier with a HLB value of between 3 and 8 is contained as the emulsifier.

9. A preparation according to claim 7, wherein a non-ionic emulsifier is included.

10. A preparation according to one of the preceding claims, wherein a co-solvent is contained in the aqueous phase.

11. A preparation according to one of the preceding claims, wherein a polymeric film-forming agent is additionally included.

12. A preparation according to one of the preceding claims, wherein an aqueous polymer dispersion with a solids content of 10 to 70% by weight is contained as the film-forming agent.

13. A preparation according to one of the preceding claims, wherein the film-forming agent is present in a proportion of 0 to 50% by weight.

14. A preparation according to one of the preceding claims, wherein a colouring agent, in particular an inorganic and/or organic dye and/or a pigment is additionally included.

15. A preparation according to one of the preceding claims, wherein the gas included is air, a protective gas selected from nitrogen, helium, neon, argon, krypton, xenon, carbon dioxide, and/or dinitrogen monoxide, a hydrocarbon gas, an ether and/or oxygen or ozone.

16. A preparation according to one of the preceding claims, which comprises 20 to 40 % by volume of a gas.

17. A preparation according to one of the preceding claims, wherein the foam has bubbles of a mean size of 0.005 to 0.5 mm.

18. A preparation according to one of the preceding claims, wherein the final preparation is of a density in the range of 0.7 to 0.9.

19. A process for the production of a foam-like cosmetic preparation as defined in claim 1, wherein
a) a continuous phase is produced by the solvent or solvents of the continuous phase being mixed with a thickening agent at ambient temperature,
b) a discontinuous phase which contains a structuring agent is produced, to which an emulsifier is optionally added,
c) the continuous and the discontinuous phase are heated to a temperature above the lower critical temperature of the thickening system and mixed, an emulsion being produced, and
d) the emulsion is agitated until it has cooled down.

20. A process according to claim 19, wherein an aqueous phase is prepared as the continuous phase and an oil phase is prepared as the discontinuous phase.

21. A process according to claim 20, wherein an O/W emulsion is produced, by
a) producing an aqueous phase by mixing water with a thickening agent at ambient temperature,
b) producing an oil phase by a procedure whereby wax is melted optionally with oils and fats, an emulsifier is added to the wax phase, the oil phase is added to the aqueous phase and an emulsion is produced, and the emulsion is agitated with the addition of gas until it has cooled down.

22. A process according to one of claims 20 to 21, wherein after production of the emulsion gas is incorporated by agitation.

23. A process according to one of claims 20 to 22, wherein 20 to 40% by volume of gas is introduced.

24. A process according to one of claims 20 to 23, wherein after formation of the emulsion the temperature-sensitive constituents are added.

25. A process according to one of claims 20 to 24, wherein a thickening agent is added to the continuous phase in such an amount that the emulsion obtained has a gel network temperature of ≥ 60°C.

26. A process according to one of claims 20 to 25, wherein the agitation rate at which the emulsion is agitated upon cooling is adjusted such that pores of a predetermined size are produced.

27. A process according to claim 26, wherein the agitation rate is adjusted such that pores of a size in the range of 0.005 to 0.5 mm are produced.

## Patentansprüche

1. Schaumartige kosmetische Zubereitung in Form einer Öl-in-Wasser-Emulsion, die eine kontinuierliche Phase, eine diskontinuierliche Phase, und ein Gas aufweist, wobei die kontinuierliche Phase ein Verdickungssystem aufweist, das durch Wasser oder ein wässriges Medium und ein Verdickungsmittel gebildet ist, wobei der Anteil des Verdickungsmittels in dem Verdickungssystem 0,5 bis 5 Gewichtsprozent beträgt, und wobei die diskontinuierliche Phase ein Strukturierungsmittel aufweist, wobei das Verdickungssystem eine Mischungslücke mit einer unteren kritischen Temperatur zwischen 35 und 80°C hat.

2. Zubereitung nach Anspruch 1, wobei das wässrige Medium eine Mischung aus Wasser und einem hydrophilen Lösungsmittel ist.

3. Zubereitung nach Anspruch 1 oder 2, wobei das wässrige Medium ein Gemisch aus Wasser und einwertigen Alkoholen, welche aus Ethanol und Propanol ausgewählt sind, oder mehrwertigen Alkoholen ist, welche aus Propandiolen, Butylenglykolen, Hexylenglykolen, Dipropylenglykol, Tripropylenglykol, Glycerin, Diglycerin, Sorbit, Mannit, Xylit und Alditol ausgewählt sind.

4. Zubereitung nach Anspruch 1, wobei das Verdickungssystem durch ein wässriges Medium und ein Cellulose-Derivat gebildet ist.

5. Zubereitung nach einem der vorhergehenden Ansprüche, wobei die diskontinuierliche Phase eine Ölphase ist, die aus fettartigen, ölartigen und/oder wachsartigen Substanzen besteht.

6. Zubereitung nach einem der vorhergehenden Ansprüche, wobei die Ölphase 20 bis 100% Wachs und 0 bis 80% Öl und/oder Fett aufweist.

7. Zubereitung nach einem der vorhergehenden Ansprüche, wobei ein Emulgator in der Emulsion enthalten ist.

8. Zubereitung nach einem der vorhergehenden Ansprüche, wobei ein Emulgator mit einem HLB-Wert von zwischen 3 und 8 als Emulgator enthalten ist.

9. Zubereitung nach Anspruch 7, wobei ein nicht-ionischer Emulgator enthalten ist.

10. Zubereitung nach einem der vorhergehenden Ansprüche, wobei ein Co-Lösungsmittel in der wässrigen Phase enthalten ist.

11. Zubereitung nach einem der vorhergehenden Ansprüche, wobei ein polymerer Filmbildner zusätzlich enthalten ist.

12. Zubereitung nach einem der vorhergehenden Ansprüche, wobei eine wässrige Polymerdispersion mit einem Feststoffgehalt von 10 bis 70 Gewichtsprozent als Filmbildner enthalten ist.

13. Zubereitung nach einem der vorhergehenden Ansprüche, wobei der Filmbildner in einem Anteil von 0 bis 50 Gewichtsprozent anwesend ist.

14. Zubereitung nach einem der vorhergehenden Ansprüche, wobei ein Färbemittel, insbesondere ein anorganischer und/oder organischer Farbstoff und/oder ein Pigment zusätzlich enthalten ist.

15. Zubereitung nach einem der vorhergehenden Ansprüche, wobei das enthaltene Gas Luft, ein Schutzgas, das aus Stickstoff, Helium, Neon, Argon, Krypton, Xenon, Kohlendioxid und/oder Distickstoffmonoxid ausgewählt ist, ein Kohlenwasserstoffgas, ein Ether und/oder Sauerstoff oder Ozon ist.

16. Zubereitung nach einem der vorhergehenden Ansprüche, die 20 bis 40 Volumenprozent eines Gases aufweist.

17. Zubereitung nach einem der vorhergehenden Ansprüche, wobei der Schaum Blasen einer mittleren Größe von 0,005 bis 0,5 mm hat.

18. Zubereitung nach einem der vorhergehenden Ansprüche, wobei die fertige Zubereitung eine Dichte im Bereich von 0,7 bis 0,9 hat.

19. Verfahren zur Herstellung einer wie in Anspruch 1 definierten schaumartigen kosmetischen Zubereitung, wobei
a) eine kontinuierliche Phase durch Mischen des Lösungsmittels oder der Lösungsmittel der kontinuierlichen Phase mit einem Verdickungsmittel bei Umgebungstemperatur hergestellt wird,
b) eine diskontinuierliche Phase, die ein Strukturierungsmittel enthält, hergestellt wird, zu der gegebenenfalls ein Emulgator zugegeben wird,
c) die kontinuierliche und diskontinuierliche Phase auf eine Temperatur oberhalb der unteren kritischen Temperatur des Verdickungssystems erwärmt und gemischt werden, wodurch eine Emulsion hergestellt wird, und
d) die Emulsion gerührt wird, bis sie abgekühlt ist.

20. Verfahren nach Anspruch 19, wobei eine wässrige Phase als kontinuierliche Phase hergestellt und eine Ölphase als diskontinuierliche Phase hergestellt wird.

21. Verfahren nach Anspruch 20, wobei eine Öl-in-Wasser-Emulsion hergestellt wird, indem
a) eine wässrige Phase durch Mischen von Wasser mit einem Verdickungsmittel bei Umgebungstemperatur hergestellt wird,
b) eine Ölphase durch ein Verfahren hergestellt wird, bei dem Wachs gegebenenfalls mit Ölen und Fetten geschmolzen wird, ein Emulgator zu der Wachsphase hinzugefügt wird, die Ölphase zu der wässrigen Phase hinzugefügt und eine Emulsion hergestellt wird, und die Emulsion unter Zugabe von Gas gerührt wird, bis sie abgekühlt ist.

22. Verfahren nach einem der Ansprüche 20 bis 21, wobei nach der Herstellung der Emulsion Gas durch Rühren eingearbeitet wird.

23. Verfahren nach einem der Ansprüche 20 bis 22, wobei 20 bis 40 Volumenprozent an Gas eingebracht wird.

24. Verfahren nach einem der Ansprüche 20 bis 23, wobei nach der Bildung der Emulsion die temperaturempfindlichen Bestandteile zugegeben werden.

25. Verfahren nach einem der Ansprüche 20 bis 24, wobei ein Verdickungsmittel zu der kontinuierlichen Phase in einer solchen Menge zugegeben wird, dass die erhaltene Emulsion eine Gelnetzwerkstemperatur von ≥ 60°C hat.

26. Verfahren nach einem der Ansprüche 20 bis 25, wobei die Rührstufe, bei der die Emulsion beim Abkühlen gerührt wird, so eingestellt wird, dass Poren einer vorbestimmten Größe hergestellt werden.

27. Verfahren nach Anspruch 26, wobei die Rührstufe so eingestellt wird, dass Poren einer Größe im Bereich von 0,005 bis 0,5 mm hergestellt werden.

## Revendications

1. Préparation cosmétique de type mousse sous la forme d'une émulsion huile-dans-l'eau, comprenant une phase continue, une phase discontinue, et un gaz, dans laquelle la phase continue comprend un système épaississant qui est formé par de l'eau ou un milieu aqueux et un agent épaississant, où la proportion de l'agent épaississant dans le système épaississant est comprise entre 0,5 et 5 % en masse, et où la phase discontinue comprend un agent structurant, où le système épaississant présente une lacune de miscibilité avec une température critique inférieure comprise entre 35 et 80 °C.

2. Préparation selon la revendication 1, dans laquelle le milieu aqueux est un mélange d'eau et d'un solvant hydrophile.

3. Préparation selon la revendication 1 ou 2, dans laquelle le milieu aqueux est un mélange d'eau et d'alcools monovalents sélectionnés parmi l'éthanol et le propanol, ou d'alcools polyvalents sélectionnés parmi des propanediols, des butylèneglycols, des héxylèneglycols, du dipropylèneglycol, du tripropylèneglycol, de la glycérine, de la diglycérine, du sorbitol, du mannitol, du xylitol et de l'alditol.

4. Préparation selon la revendication 1, dans laquelle le système épaississant est formé par un milieu aqueux et un dérivé de cellulose.

5. Préparation selon l'une des revendications précédentes, dans laquelle la phase discontinue est une phase huileuse qui est formée de substances graisseuses, huileuses et/ou cireuses.

6. Préparation selon l'une des revendications précédentes, dans laquelle la phase huileuse comprend 20 à 100 % de cire et 0 à 80 % d'huile et/ou de graisse.

7. Préparation selon l'une des revendications précédentes, dans laquelle l'émulsion contient un émulsifiant.

8. Préparation selon l'une des revendications précédentes, dans laquelle est présent en tant qu'émulsifiant un émulsifiant présentant un rapport hydrophilelipophile (valeur HLB) compris entre 3 et 8.

9. Préparation selon la revendication 7, dans laquelle est inclus un émulsifiant non ionique.

10. Préparation selon l'une des revendications précédentes, dans laquelle un co-solvant est contenu dans la phase aqueuse.

11. Préparation selon l'une des revendications précédentes, dans laquelle est en outre inclus un agent filmogène polymère.

12. Préparation selon l'une des revendications précédentes, dans laquelle est contenu en tant qu'agent filmogène une dispersion polymère aqueuse présentant une teneur en solides comprise entre 10 et 70 % en masse.

13. Préparation selon l'une des revendications précédentes, dans laquelle l'agent filmogène est présent dans une proportion comprise entre 0 et 50 % en masse.

14. Préparation selon l'une des revendications précédentes, dans laquelle est en outre inclus un agent colorant, en particulier un colorant inorganique et/ou organique et/ou un pigment.

15. Préparation selon l'une des revendications précédentes, dans laquelle le gaz inclus est de l'air, un gaz protecteur sélectionné parmi l'azote, l'hélium, le néon, l'argon, le crypton, le xénon, le dioxyde de carbone, et/ou le monoxyde de diazote, un gaz hydrocarboné, un éther et/ou l'oxygène ou l'ozone.

16. Préparation selon l'une des revendications précédentes, qui comprend 20 à 40 % en volume d'un gaz.

17. Préparation selon l'une des revendications précédentes, dans laquelle la mousse a des bulles qui présentent une taille moyenne comprise entre 0,005 et 0,5 mm.

18. Préparation selon l'une des revendications précédentes, dans laquelle la préparation finale présente une densité dans la plage de 0,7 à 0,9.

19. Procédé de production d'une préparation cosmétique de type mousse telle que définie dans la revendication 1, dans lequel
a) on produit une phase continue en mélangeant le solvant ou les solvants de la phase continue avec un agent épaississant à la température ambiante,
b) on produit une phase discontinue qui contient un agent structurant, à laquelle on ajoute en option un émulsifiant,
c) on chauffe la phase continue et la phase discontinue à une température supérieure à la température critique inférieure du système d'épaississant et on mélange, ce qui produit une émulsion, et
d) on agite l'émulsion jusqu'à son refroidissement.

20. Procédé selon la revendication 19, dans lequel la phase aqueuse est préparée en tant que phase continue, et une phase huileuse est préparée en tant que phase discontinue.

21. Procédé selon la revendication 20, dans lequel on produit une émulsion huile-dans-l'eau par
a) production d'une phase aqueuse en mélangeant de l'eau avec un agent épaississant à la température ambiante,
b) production d'une phase huileuse par une procédure selon laquelle de la cire est fondue, en option avec des huiles et des graisses, un émulsifiant est ajouté à la phase cireuse, la phase huileuse est ajoutée à la phase aqueuse et une émulsion est produite, puis l'émulsion est agitée avec addition de gaz jusqu'à son refroidissement.

22. Procédé selon l'une des revendications 20 à 21, dans lequel, suite à la production de l'émulsion, on incorpore du gaz par agitation.

23. Procédé selon l'une des revendications 20 à 22, dans lequel 20 à 40 % en volume de gaz sont introduits.

24. Procédé selon l'une des revendications 20 à 23, dans lequel, suite à la formation de l'émulsion, on ajoute les constituants sensibles à la température.

25. Procédé selon l'une des revendications 20 à 24, dans lequel on ajoute un agent épaississant à la phase continue dans une quantité telle que l'émulsion obtenue a une température de réticulation en gel ≥ 60 °C.

26. Procédé selon l'une des revendications 20 à 25, dans lequel la vitesse d'agitation à laquelle l'émulsion est agitée lors du refroidissement est ajustée de façon à produire une taille de pores prédéterminée.

27. Procédé selon la revendication 26, dans lequel la vitesse d'agitation est ajustée de façon telle que sont produits des pores dont la taille est comprise dans la plage de 0,005 à 0,5 mm.
